# FASCICULE DE BREVET EUROPEEN

(11) **EP 2 774 669 B1**
(45) Date de publication et mention de la délivrance du brevet: **29.08.2018**
(21) Numéro de dépôt: 14157729.6
(22) Date de dépôt: 04.03.2014
(51) Int. Cl.: B01J 13/10, A61F 13/15, A61F 13/49, A61F 13/20

(54) **ARTICLE ABSORBANT**
SAUGFÄHIGER ARTIKEL
ABSORBENT ARTICLE

(30) Priorité: 05.03.2013 FR 1351969
(43) Date de publication de la demande: 10.09.2014
(73) Titulaire: Microcapsules Technologies, 45390 Puiseaux (FR)
(72) Inventeur: Habar, Gérard, 77780 Bourron-Marlotte (FR)
(74) Mandataire: Nony

(56) Documents cités:
- WO-A2-03/020240
- FR-A1- 2 787 024
- US-A1- 2003 120 224
- US-A1- 2003 120 225

## Description

### DOMAINE DE L'INVENTION

La présente invention concerne le domaine des articles absorbants, notamment d'hygiène, et en particulier d'hygiène personnelle. La présente invention concerne plus particulièrement un article absorbant, notamment d'hygiène, comprenant, de manière séparée, d'une part, des microcapsules spécifiques chargées en au moins un actif et, d'autre part, au moins un agent de libération apte à stimuler la libération de l'actif présent au sein desdites microcapsules lors de la mise en contact de l'article absorbant avec un milieu aqueux.

### ART ANTERIEUR

Les articles absorbants d'hygiène personnelle sont bien connus. A titre d'exemples typiques de tels articles absorbants, on peut notamment citer les serviettes hygiéniques, les protège-slips, les tampons, les articles pour adultes incontinents, les couches pour bébés, etc...

Ces articles sont couramment utilisés pour absorber et retenir les fluides corporels et les autres exsudats excrétés par le corps humain, tels que l'urine et les menstruations.

Ces articles absorbants d'hygiène personnelle peuvent toutefois conduire à l'apparition d'irritations et/ou d'allergies, notamment chez les personnes ayant une peau sensible, et/ou, de par la présence des fluides et/ou exsudats corporels, être perçus comme malodorant.

Par conséquent, de nombreuses méthodes et matériaux pour maîtriser et réduire ces irritations, allergies et/ou mauvaises odeurs dans des articles absorbants ont été développés.

Eu égard en particulier à la problématique des mauvaises odeurs, des matières parfumées sont largement utilisées à cette fin, ainsi que des ingrédients tels que la silice ou des zéolites qui sont capables de piéger une partie des molécules génératrices de mauvaises odeurs.

Un inconvénient couramment observé est la manifestation permanente de l'odeur de parfum au niveau de l'article absorbant et donc, avant usage dudit article, ce qui, pour des raisons évidentes, peut ne pas être acceptable pour certains consommateurs.

En outre, de par le contact continu de l'article absorbant avec le milieu extérieur, un autre inconvénient rencontré est une altération de la puissance olfactive, voire une modification, des matières parfumées avec le temps.

Des technologies de contrôle des mauvaises odeurs qui ne donnent pas une odeur perceptible à l'article absorbant avant son utilisation ont déjà été proposées.

A ce titre, peuvent notamment être cités les documents WO 94/22500, WO 94/22501, WO 2005/120412, US 6,369,290, US 5,733,272, US 5,429,628 ou WO 2012/087891. US 2003/120224 A1 décrit un tampon comprenant des microcapsules de dextran chargées avec un principe actif et un tensioactif ionique. Enfin, un autre inconvénient est la vitesse de libération des matières parfumées qui, parfois, peut-être insuffisante si bien qu'un dégagement malodorant est malgré tout ressenti.

Bien que des articles absorbants d'hygiène personnelle à ce jour sur le marché puissent être jugés satisfaisants à l'égard des aspects évoqués ci-dessus, la mise en oeuvre d'articles absorbants d'hygiène personnelle présentant des caractéristiques encore améliorées sur ces aspects, en particulier en termes de rapidité de libération de l'actif considéré, demeure un objectif constant.

### RESUME DE L'INVENTION

Les inventeurs ont constaté que la mise en oeuvre d'un actif encapsulés au sein de microcapsules spécifiques, en association avec un actif secondaire (désigné, dans la suite de la présente demande, « agent de libération ») dédié à stimuler la libération dudit actif microencapsulé dans des conditions particulières, conduit à une alternative intéressante aux méthodes proposées à ce jour.

Ainsi, selon un premier de ses aspects, la présente invention concerne un article absorbant comprenant, sous une forme immobilisée:
a) des microcapsules formées à raison d'au moins 25 % en poids, de préférence d'au moins 50 % en poids, par rapport au poids total en polymère(s) formant les microcapsules d'un matériau dérivant d'au moins un polymère naturel, d'un de ses analogues protéiniques, ou d'un de ses dérivés, et chargées en au moins un actif, et
b) au moins un agent de libération, apte à stimuler la dissolution des microcapsules lors de la mise en contact de l'article absorbant avec un milieu aqueux et choisi parmi les enzymes, les tensioactifs hydrosolubles à température ambiante et pression atmosphérique, et leurs mélanges,
les microcapsules a) et l'agent de libération b) étant présents dans ledit article sous une forme isolée l'une de l'autre.

Par ailleurs, la présente divulgation concerne un article absorbant comprenant, sous une forme immobilisée :
a) des microcapsules au moins partiellement réticulées et chargées en au moins un actif, et
b) au moins un agent de libération, apte à stimuler la libération de l'actif encapsulé lors de la mise en contact de l'article absorbant avec un milieu aqueux, et choisi parmi les enzymes, les tensioactifs hydrosolubles à température ambiante et pression atmosphérique, et leurs mélanges,
les microcapsules a) et l'agent de libération b) étant présents dans ledit article sous une forme isolée l'une de l'autre.

Il est entendu que l'article absorbant tel que défini ci-dessus reproduit toutes ces spécificités sous sa forme native, c'est-à-dire avant usage.

Ainsi, par « avant usage », ou encore « prêt à l'emploi », au sens de la présente invention, on entend désigner un article absorbant dans son état tel qu'il se trouve avant sa mise en contact avec le(s) fluide(s) corporel(s) et/ou exsudats excrétés par le corps humain à absorber. Par « dérivant d'au moins un polymère naturel, d'un de ses analogues protéiniques, ou d'un de ses dérivés », on entend désigner au sens de la présente invention, le fait que le matériau peut être constitué d'un ou de plusieurs polymère(s) en tant que tel, mais également d'un/de composé(s) obtenu(s) à l'issue de la réticulation partielle ou totale susceptible d'être considérée dans la technique de fabrication des microcapsules selon l'invention, comme décrit ci-après.

Par « microcapsules au moins partiellement réticulées », au sens de la présente divulgation on entend désigner des microcapsules formées au moins en partie d'au moins un polymère présent seulement en partie sous une forme réticulée. En d'autres termes, une partie dudit polymère constituant les microcapsules y est présente sous une forme non réticulée.

Ainsi, les inventeurs ont aujourd'hui constaté que l'agencement, dans un même article, de microcapsules particulières chargées en actif(s) et d'un agent de libération réactif à l'égard du matériau constituant en tout ou partie ces microcapsules s'avère particulièrement avantageux à plusieurs titres. En effet, la présence de l'agent de libération conduit à une nette stimulation de la vitesse de libération de l'actif microencapsulé et donc, à une manifestation sous bref délai, voire immédiate, de l'effet recherché attaché à cet actif spécifique.

Tout d'abord, l'article autorise une libération contrôlée de l'actif considéré. En effet, ce dernier n'est libéré qu'une fois ledit article en contact avec le milieu aqueux.

Qui plus est, de par le caractère microencapsulé dudit actif, ce dernier peut conserver son intégrité, et donc son efficacité, plus longtemps.

Par « article absorbant », au sens de la présente invention, on entend désigner tout dispositif apte à absorber et retenir les milieux liquides, notamment physiologiques, tels que les exsudats ou fluides corporels tels que les larmes, le mucus nasal, l'urine, la sueur, les menstruations, et les matières fécales.

Ainsi, des exemples d'articles absorbants selon l'invention peuvent comprendre les couches, les couches pour enfant, les vêtements pour adultes incontinents et les vêtements d'hygiène féminine tels que des serviettes hygiéniques, les protège-slips, les tampons hygiéniques, les dispositifs interlabiaux, les coussinets hémorroïdaires, etc.

Cependant, l'agencement selon l'invention est tel qu'il peut également être considéré dans des articles absorbants tels que par exemple des éponges, des lingettes, des mouchoirs, etc...

Selon un autre de ses aspects, la présente invention concerne l'utilisation d'un article tel que défini ci-dessus, pour maîtriser et/ou réduire les mauvaises odeurs des fluides corporels et/ou exsudats excrétés par le corps humain, en particulier l'urine et les menstruations.

Selon encore un autre de ses aspects, la présente invention concerne l'utilisation d'un article tel que défini ci-dessus, pour maîtriser et/ou réduire les irritations, inflammations et/ou infections liées audit article et/ou aux fluides corporels et/ou exsudats excrétés par le corps humain.

### DESCRIPTION DETAILLEE DE L'INVENTION

### Microcapsules

Au sens de l'invention, une « microcapsule » désigne une entité matérielle de taille micrométrique présentant une architecture dite coeur/écorce et dont l'écorce ou enveloppe entoure le coeur, dit encore noyau, dans lequel est encapsulé un ou plusieurs actifs.

Ce coeur peut être figuré par une cavité ou être de nature matricielle.

L'écorce entourant le coeur peut être de même nature ou non que le coeur sous réserve qu'elle soit propice à l'encapsulation d'actif(s).

Ainsi, des microcapsules peuvent être creuses (vésiculaires) ou pleines (matricielles).

A titre illustratif des architectures de microcapsules déjà connues, on peut notamment citer les liposomes ; les microsphères ou microbilles qui sont des particules constituées d'un réseau de macromolécules, réseau dans lequel est piégée la substance active sous forme de molécules, de particules solides micrométriques ou de fines gouttelettes de solution ; ou les microcapsules qui sont des particules creuses entourées d'une paroi plus ou moins rigide.

Les microcapsules plus particulièrement considérées selon l'invention sont des microparticules « à réservoir », c'est-à-dire présentant une architecture de type coeur/écorce ou « *core*/*shell* ».

Pour des raisons évidentes, la nature des microcapsules selon l'invention est telle qu'elle est compatible avec la destination d'un article absorbant selon l'invention. En d'autres termes, les microcapsules sont réalisées dans des matériaux de préférence inertes à l'égard de l'utilisateur, afin d'éviter tout effet indésirable tel que par exemples des phénomènes d'allergies et/ou d'irritations.

Egalement, la nature des microcapsules selon l'invention est telle qu'elle est inerte vis-à-vis des actifs dédiés à y être microencapsulé.

Enfin, la nature des microcapsules selon l'invention est telle qu'elle est réactive à l'égard de l'agent de libération associé.

Au sens de l'invention, « réactive » signifie que la mise en contact des microcapsules ou, tout du moins, du matériau formant la paroi externe de cette microcapsule ou encore d'au moins un des matériaux formant cette paroi, induit une perte de cohésion de la surface externe (ou paroi) des microcapsules sous bref délai, voire immédiatement, provoquant ainsi la libération de l'actif microencapsulé.

Cette perte de cohésion peut se manifester de plusieurs manières, à savoir rupture, gonflement, altération ou destruction.

Par « rupture », « altération » ou « destruction » des microcapsules, on entend donc au sens de la présente invention la perte de cohésion de la paroi desdites microcapsules, qui peut s'opérer selon au moins l'un des mécanismes non limitatifs suivants, à savoir solubilisation, décomposition, désintégration, dissociation, séparation, dissolution, fracturation, gonflement, ou augmentation de la porosité, voire une extraction de l'actif par l'agent de libération.

Ce mécanisme peut dépendre notamment, d'une part, de la nature d'au moins un des matériaux formant la microcapsule et, d'autre part, de la nature de l'agent de libération associé.

Plus précisément, cette réactivité se traduit par une stimulation de la libération de l'actif encapsulé.

Par « stimulation », au sens de la présente invention, on entend qualifier une augmentation de la vitesse de perte de cohésion telle que définie ci-dessus en présence d'au moins un agent de libération véhiculé par un milieu aqueux, et donc de libération dudit actif encapsulé, comparativement aux microcapsules selon l'invention mises en contact uniquement avec le milieu aqueux, c'est à dire en absence de l'agent de libération.

Cette augmentation de vitesse de libération de l'actif microencapsulé, généralement significative, peut varier selon le degré de réticulation de la paroi des microcapsules et par le choix et le dosage de l'agent de libération.

Le(s) matériau(x) utilisé(s) pour la fabrication de microcapsules selon l'invention et le degré de réticulation considéré sont tels que lesdites microcapsules sont de préférence peu sensibles à l'eau ou, en d'autres termes, manifestent avantageusement une résistance mécanique prolongée à l'eau.

En d'autres termes, leur altération, et donc la libération des actifs microencapsulés, au contact d'un milieu aqueux non supplémenté en un agent de libération selon l'invention ne pourra se manifester qu'au terme d'un délai significativement plus prolongé comparativement au délai constaté au contact de ce même milieu aqueux mais supplémenté en un tel agent de libération.

Comme indiqué précédemment, les microcapsules selon l'invention sont formées à raison d'au moins 25 % en poids, de préférence d'au moins 50 % en poids, par rapport au poids total en polymère(s) formant les microcapsules d'un matériau dérivant d'au moins un polymère naturel, d'un de ses analogues protéiniques, ou d'un de ses dérivés.

Le choix des polymères naturels est avantageux au vu de leur caractère hydrophile, voire même soluble dans l'eau, ce qui conduit à faciliter la fabrication des microcapsules, via notamment les techniques des microencapsulations définies ci-après.

En effet, les microcapsules constituées de matériaux « synthétiques », tels que par exemple la mélamine formaldéhyde, l'urée formol, le polyuréthane, les acryliques, le polyamide ou le silicone sont des microcapsules très réticulées et donc peu sensibles aux produits chimiques, en particulier à l'eau et ce, même en présence d'un agent de libération selon l'invention. En conséquence, la mise en oeuvre de microcapsules constituées uniquement de matériaux « synthétiques » conduirait à une libération de l'actif microencapsulé trop lente et ce, malgré l'utilisation d'un agent de libération selon l'invention.

Un autre avantage attaché aux polymères naturels est, qu'une fois les microcapsules formées, notamment via une des techniques de microencapsulation définies ci-après, leur degré de réticulation, et donc de sensibilité à l'eau, peut-être adapté très facilement selon la nature et la quantité en agent(s) réticulant.

L'origine naturelle des polymères considérés dans la présente invention à partir desquels le matériau des microcapsules dédié à réagir avec l'agent de libération est susceptible de dériver est d'autant plus avantageuse qu'elle répond à une demande des consommateurs en ce sens.

De préférence, à l'exception des actifs microencapsulés, l'ensemble des composés formant les microcapsules selon l'invention sont d'origine naturelle.

La nature du polymère naturel peut notamment être conditionnée par la technique de microencapsulation considérée, comme indiqué ci-après.

Le polymère naturel est de préférence majoritairement linéaire. Cette caractéristique est avantageuse en ce qu'elle permet de faire varier la sensibilité à l'eau desdites microcapsules.

A titre de polymère naturel convenant à la confection de microcapsules conformes à l'invention, peuvent notamment être citées les polymères naturels d'origine animale ou végétale.

De préférence, les polymères naturels selon l'invention peuvent être choisis parmi les polymères naturels amphotères ou cationisables.

De manière encore plus préférés, les polymères naturels selon l'invention peuvent être choisis parmi la gélatine, la chitine, les polyosides ou polysaccharides choisis parmi la gomme arabique, la gomme adragante, la gomme karaya, les galactomannanes issus de certaines graines, comme le guar ou la caroube, la gomme tara, les pectines situées dans les parois cellulaires et les ciments intracellulaires des végétaux, les polyosides issus de fermentation bactérienne, la gomme de xanthane, la gomme gellane, la gomme scléroglucanne, les extraits d'algues, notamment d'algues rouges tels que l'agar-agar et les carraghénanes, ou d'algues brunes tels que les alginates, un de leurs analogues protéiniques, ou un de leurs dérivés, et leurs mélanges.

Par « gélatine », au sens de la présente invention, on entend désigner tout produit protéiné obtenu par hydrolyse partielle du collagène extrait de la peau comme la peau du porc (cochon), des os, des cartilages, des ligaments etc... Plus particulièrement, la gélatine résulte d'une cassure des liaisons moléculaires entre les brins de collagène, obtenue par ébullition prolongée puis solidification par refroidissement. Mélangée à de l'eau, elle forme un gel colloïdal semi-solide.

Par « chitine », au sens de la présente invention, on entend désigner un polysaccharide issu de la polymérisation de N-acétylglucosamine liés entre eux par une liaison du type β-1,4. C'est une molécule structurale importante chez plusieurs taxons d'eucaryotes, notamment les champignons et les arthropodes.

Par « analogue protéinique», on entend désigner, au sens de la présente invention, tout produit protéiné présentant des propriétés physiques et chimiques similaires à un polymère naturel selon l'invention et présentant un comportement similaire vis-à-vis d'un milieu aqueux et d'un agent de libération dans le cadre d'un article absorbant selon la présente invention en termes de perte de cohésion, voire de désintégration.

A titre d'analogue protéinique d'un polymère naturel conforme à l'invention, on peut notamment citer les protéines végétales telles celles extraites du soja, du lupin ou du blé.

Par « dérivés », on entend désigner, au sens de la présente invention, tout produit présentant des propriétés physiques et chimiques similaires au polymère naturel à partir duquel il est obtenu et présentant un comportement similaire vis-à-vis d'un milieu aqueux et d'un agent de libération dans le cadre d'un article absorbant selon la présente invention en termes de perte de cohésion, voire de désintégration.

A titre de dérivé d'un polymère naturel conforme à l'invention, on peut notamment citer le chitosan, les dérivés de la cellulose tels que la carboxyméthylcellulose et les amidons modifiés.

Le chitosane ou chitosan est un polyoside composé de la distribution aléatoire de D-glucosamine liée en β-(1-4) (unité désacétylée) et de N-acétyl-D-glucosamide (unité acétylée). Il est produit par désacétylation chimique (en milieu alcalin) ou enzymatique de la chitine.

De préférence, le polymère naturel amphotère est figuré par la gélatine.

Le(s) polymère(s) naturel(s) amphotère(s) ou cationisable(s), un de ses/leurs dérivés ou un de ses/leurs analogues protéiniques, de préférence la gélatine, ont généralement une force en gelée allant de 120 bloom à 300 bloom, de préférence de 130 bloom à 200 bloom, et mieux de 150 à 180 bloom.

Par ailleurs, au vu de ce qui précède, le matériau dérivant d'au moins un polymère naturel, d'un de ses analogues protéiniques, ou d'un de ses dérivés, et dédié à réagir avec l'agent de libération peut constituer l'unique composante des microcapsules.

Cependant, des microcapsules conformes à la présente invention peuvent également mettre en oeuvre ce matériau en mélange avec d'autres composés, dits composés annexes.

Peuvent notamment être cités à titre de tels composés annexes les polyacides synthétiques tels les acides polyacryliques ou méthacryliques et leur copolymères, l'acide polyaspartique, les copolymères de polyvinylpirolidone et d'anhydride maléique tels les gantrez.

Le choix et la proportion de ce ou ces composés annexes sont bien entendu ajustés pour ne pas porter préjudice aux propriétés recherchées attachées aux microcapsules selon l'invention. Egalement, le choix et la proportion de ce ou ces composés annexes peuvent être conditionnés par la technique de microencapsulation considérée. Ces ajustements relèvent clairement des compétences de l'homme de l'art.

De préférence, le matériau dérivant d'au moins un polymère naturel, d'un de ses analogues protéiniques, ou d'un de ses dérivés, peut former les microcapsules à raison d'au moins 50 % en poids, de préférence d'au moins 60 % en poids, plus particulièrement d'au moins 70 % en poids, et mieux d'au moins 80 % en poids, par rapport au poids total en polymère(s) formant les microcapsules.

Comme il ressort de ce qui précède, les microcapsules considérées selon l'invention sont chargées en un ou plusieurs actifs d'intérêt.

Ceci peut être réalisé par toute technique de microencapsulation connue sous réserve qu'elle soit compatible avec la nature du/des matériaux considéré(s) pour les microcapsules et l'/les actif(s) considéré(s).

Par « microencapsulation », on entend donc désigner, au sens de l'invention, tout procédé par lequel on enferme un produit, solide, liquide ou pâteux, dans des microcapsules.

Les principaux procédés pour réaliser l'encapsulation d'au moins un actif dans des microcapsules sont généralement des procédés assurant conjointement la confection des microcapsules et l'encapsulation de l'actif considéré au sein desdites microcapsules.

A titre illustratif des techniques d'encapsulation mettant en oeuvre des polymères naturels tels que cités précédemment et pouvant être considérées selon l'invention, peuvent notamment être citées l'émulsion suivie d'une évaporation ou d'une extraction du solvant, la double émulsion évaporation/extraction de solvant, l'atomisation (ou « *spray drying* »), le prilling, la coacervation simple ou complexe, la formation de films multicouches autoassemblés LBL (Layer by layer).

La polymérisation *in situ* de résines synthétiques telles les résines mélamine, l'urée formol, les polyamides, les acryliques, les polyuréthanes peut également être envisagée par incorporation de polymères naturels.

Les polymères naturels privilégiés pour la technique de coacervation complexe peuvent être les polymères naturels amphotères ou cationisables, de préférence choisis parmi la gélatine, le chitosan, les dérivés de cellulose, la gomme arabique, les alginates, et leurs mélanges.

Les polymères naturels privilégiés pour la technique de spray drying ou de prilling peuvent être les polymères naturels non ioniques, anioniques ou cationiques.

Ainsi, les polymères naturels privilégiés pour la technique de spray drying peuvent être les dérivés de cellulose, les dérivés d'amidon, et leurs mélanges.

Les polymères naturels privilégiés pour la technique de prilling peuvent être les alginates.

Le choix final du type de technique et de la nature des microcapsules dépend de l'application et de facteurs tels que la dimension particulaire, l'épaisseur de la paroi, l'imperméabilité, la stabilité thermique, la dégradabilité, la compatibilité et l'adhérence à l'environnement de l'utilisation finale.

L'actif microencapsulé selon l'invention résulte avantageusement d'une microencapsulation par coacervation, et mieux d'une microencapsulation par coacervation complexe.

La technique de microencapsulation par coacervation complexe est en effet privilégiée compte-tenu du fait qu'elle est envisageable pour la formulation de microcapsules à partir de composés d'origine naturelle, notamment la gélatine, et dont la transformation permet d'accéder à des propriétés avantageuses en termes de souplesse, de transparence, d'adhésion naturelle, de libération, de biodégradabilité, d'humidité, de compatibilité et de pH.

Outre ces avantages, l'intérêt de cette technique est de permettre l'obtention de microcapsules suffisamment réticulées pour être suffisamment étanches dans les conditions d'utilisation et par là même peu sensibles à l'eau et de permettre néanmoins la libération rapide de l'actif microencapsulé sous l'influence de l'agent de libération en présence d'un milieu aqueux. Cette technique est donc particulièrement adaptée notamment vis-à-vis d'un actif figuré par un produit volatil tel que par exemple les parfums. C'est cette technique qui permet d'obtenir également le pourcentage d'actif dans les microcapsules (« payload ») le plus élevé par rapport à leur poids total.

Plus généralement, des microcapsules résultant d'une microencapsulation par coacervation complexe sont formées par précipitation, en milieu acide, d'un complexe formé à partir d'au moins un polymère naturel, un de ses analogues protéiques, ou un de ses dérivés et d'au moins d'un polymère portant des charges anioniques.

Des polymères anioniques différents servent à améliorer le système et également faire en sorte que les microcapsules soient isolées ou se forment en grappe, tels les homopolymères et copolymères acryliques.

De préférence, le coacervat une fois déposé autour des gouttes de l'émulsion est ensuite compacté par refroidissement puis réticulé pour rendre la réaction irréversible (empêcher la redissolution dans l'eau de l'enveloppe à pH basique).

La technique de microencapsulation par coacervation complexe est notamment documentée dans les documents FR 2 718 059, FR 08 57111, US 4, 406,816 ou EP 0 674 942.

Ainsi, le procédé décrit dans le document EP 0 674 942 propose de former une suspension dans une phase aqueuse acide de particules constituées de gouttelettes d'un liquide hydrophobe, lesdites gouttelettes étant revêtues d'un coacervat formé de gélatine, d'acide polyacrylique et de carboxyméthylcellulose, de refroidir l'ensemble pour solidifier la paroi des particules, et de réticuler la gélatine pour fixer la structure et rendre le processus irréversible.

Les microcapsules selon l'invention sont donc de préférence obtenues au moyen d'une technique de microencapsulation par coacervation complexe d'au moins un polymère naturel amphotère ou cationisable et d'au moins un polymère portant des charges anioniques, le polymère portant des charges anioniques pouvant également être d'origine naturelle.

De préférence, le(s) polymère(s) portant des charges anioniques peut/peuvent être choisi(s) parmi l'acide polyacrylique, la carboxyméthylcellulose, les alginates, les polyacides synthétiques, la gomme arabique et leurs mélanges.

La réticulation peut être faite avec une intensité variable, de préférence par mise en oeuvre d'un aldéhyde, en particulier du glutaraldéhyde ou du formaldéhyde. Cette étape de réticulation peut être complétée par l'action de tanins ou autres agents de réticulation bien connus de l'homme de l'art.

Plus la réticulation est poussée, plus les microcapsules deviennent insensibles aux produits chimiques, dont l'eau, les enzymes et les tensioactifs et deviennent également étanches.

Ainsi, avantageusement, lorsque les microcapsules sont obtenues par la technique de microencapsulation par coacervation complexe, ladite technique comprend en outre, postérieurement à l'étape de coarcervation, une étape de réticulation *via* un aldéhyde, de préférence du glutaraldéhyde, l'aldéhyde pouvant de préférence représenter de 5 % à 30 % en poids, préférentiellement de 10 à 20 % en poids, par rapport au poids total en polymère(s) naturel(s) amphotère(s) ou cationisable(s).

La technique de microencapsulation considérée pour l'obtention de microcapsules conformes à l'invention peut également être l'atomisation (ou « *spray drying* »).

Cette technique de microencapsulation est plus particulièrement avantageuse pour accéder à des microcapsules, certes moins étanches que celles obtenues par la technique de microencapsulation par coacervation complexe telle que définie ci-dessus, mais dotées d'une sensibilité à l'eau améliorée, notamment en présence d'un agent de libération, de préférence un tensioactif, tel que décrit ci-après.

Pour obtenir des microcapsules selon ce mode de réalisation particulier, on pulvérise dans une tour d'atomisation l'actif à encapsuler avec au moins un dérivé d'amidon ou de cellulose ou une gomme naturelle ou synthétique en solution dans l'eau. Il en ressort un produit plus ou moins entouré d'une coque d'épaisseur variable en poudre que l'on appliquera sans présence d'eau sur un support adapté.

Les microcapsules selon l'invention peuvent être sous forme isolées (sphériques) ou agglomérées (clusters ou grappes).

Le diamètre extérieur des microcapsules, sous forme isolée ou agglomérée, peut de préférence être compris entre 0,5 µm et 1 mm, voire entre 2 et 10 µm.

En effet, trop petites, les microcapsules seront poreuses et retiendront mal l'actif. Trop grandes, elles n'auront pas la sensibilité nécessaire car la surface d'interaction avec l'agent de libération sera trop faible.

### Agent de libération

Comme indiqué précédemment, l'agent de libération est destiné à interagir avec au moins l'un des matériaux constituant les microcapsules associées dans un article conforme à l'invention, à savoir le polymère naturel, un de ses analogues protéiniques ou un de ses dérivés tels que décrits précédemment.

Pour des raisons évidentes, le choix de l'agent de libération est conditionné par le choix du matériau ou de l'un des matériaux composant l'architecture des microcapsules.

Comme précisé ci-dessus, la réactivité des microcapsules à l'égard de l'agent de libération se traduit par le fait que la mise en contact dudit agent de libération avec une microcapsule contenant ledit actif provoque une perte de cohésion de la paroi de ladite microcapsule qui peut s'opérer par solubilisation, décomposition, désintégration, dissociation, séparation, dissolution, fracturation, gonflement ou augmentation de la porosité, voire une extraction de l'actif par l'agent de libération, avec pour conséquence la libération plus ou moins rapide de l'actif encapsulé. Dans la mesure où l'agent de libération est localisé de manière séparée des microcapsules associées, cette mise en contact ne peut avantageusement se produire que lorsqu'il est véhiculé par un milieu aqueux.

Par ailleurs, pour des raisons évidentes, l'agent de libération se doit d'être inerte à l'égard de l'utilisateur, afin d'éviter tout effet indésirable tel que par exemples des phénomènes d'allergies et/ou d'irritations.

L'agent de libération peut être sous une forme liquide ou solide. Il doit pouvoir être véhiculé par un milieu aqueux vers les microcapsules afin d'assurer la libération de l'actif microencapsulé.

De préférence, l'agent de libération peut être choisi parmi les enzymes, les tensioactifs hydrosolubles à température ambiante et pression atmosphérique et leurs mélanges.

En milieu aqueux, les tensioactifs font gonfler la paroi des microcapsules et la rende poreuse. Ils vont ensuite participer à l'extraction de l'actif encapsulé qui va, sous leur influence, migrer de l'intérieur de la microcapsule vers le milieu extérieur.

L'enzyme à titre d'unique agent de libération est avantageuse lorsque l'on désire une libération contrôlée de l'actif encapsulé sur un temps prolongé. Cela concerne notamment les actifs cosmétiques, de préférence non-volatiles.

L'enzyme peut, de préférence être de type protéase ou amylase. La nature de l'enzyme est fonction de la nature du polymère naturel considéré pour la fabrication des microcapsules. Ainsi, une protéase est appropriée lorsque le polymère naturel est figuré par une protéine, en particulier la gélatine, et une amylase est appropriée lorsque le polymère naturel est figuré par un polysaccharide, en particulier l'amidon.

Par exemple, lorsque le polymère naturel est figuré par la gélatine, l'enzyme peut avantageusement être figurée par Purafect OX Enzyme (danisco US Inc. - Genencor Division).

De manière plus préférée, l'agent de libération peut être choisi parmi les tensioactifs hydrosolubles à température ambiante et pression atmosphérique, et mieux parmi les tensioactifs non ioniques, les tensioactifs anioniques, les tensioactifs amphotères, les tensioactifs cationiques, et leurs mélanges.

L'agent de libération peut de préférence être figuré par au moins un tensioactif hydrosoluble à température ambiante et pression atmosphérique choisi parmi l'oxyde de cocodiméthyl amine, l'huile de ricin hydrogénée PEG-60, les alcools en C₁₆₋₁₈ éthoxylés, les alcools gras en C₁₂₋₁₈ sulfatés, et leurs mélanges, de préférence l'huile de ricin hydrogénée PEG-60 et les alcools gras en C₁₂₋₁₈ sulfatés, et mieux les alcools gras en C₁₂₋₁₈ sulfatés.

Selon un mode de réalisation particulier, l'agent de libération est figuré par une combinaison d'au moins un tensioactif et d'au moins une enzyme tels que décrits ci-dessus.

Ce mode de réalisation particulier est avantageux en ce qu'il conduit à améliorer encore la libération de l'actif encapsulé, notamment en termes de rapidité et de quantité libérée.

Il est entendu que l'immobilisation des microcapsules et de l'agent de libération sur un article absorbant selon l'invention peut être obtenue selon différentes techniques.

Le choix de la technique d'immobilisation de l'agent de libération adéquate relève clairement des compétences de l'homme de l'art.

Elle est bien entendu susceptible de varier avec la taille des microcapsules, la nature chimique du matériau composant tout ou partie des microcapsules, la nature de l'agent de libération et la nature du/des matériaux formant l'article absorbant ou tout du moins la ou les couches de l'article absorbant sur laquelle/lesquelles les microcapsules et l'agent de libération sont dédiés à être immobilisés.

Par exemple, les techniques opératoires pour promouvoir la fonctionnalisation de l'article selon l'invention avec les microcapsules et l'agent de libération associé peuvent être de type :
- immersion de l'article, ou tout du moins la ou les couches de l'article absorbant sur laquelle/lesquelles les microcapsules ou l'agent de libération sont dédiés à être immobilisés, dans un bain contenant l'entité à immobiliser ; ou
- pulvérisation ou vaporisation de l'entité à immobiliser au niveau de la surface de l'article, ou tout du moins la ou les couches de l'article absorbant sur laquelle/lesquelles les microcapsules ou l'agent de libération sont dédiés à être immobilisés.

De préférence, l'immobilisation des microcapsules sur au moins une couche formant l'article absorbant selon l'invention requière le moins possible l'apport d'eau. A titre d'exemples de telles techniques, peut notamment être cité la pulvérisation, le saupoudrage de capsules sèches, le dépôt par couchage avec un cylindre. Dans tous les cas, on peut ajouter un liant.

L'immobilisation de l'agent de libération peut avantageusement être obtenue par pulvérisation sous forme liquide (solution dans l'eau) sur au moins une des couches formant un article absorbant selon l'invention puis séchage de ladite couche.

De préférence, l'agent de libération est immobilisé au niveau d'une couche distincte de celle sur laquelle les microcapsules sont immobilisées.

### Actif(s) présent(s) au sein des microcapsules

Les microcapsules selon l'invention sont propices à l'encapsulation d'une grande variété d'actif.

La nature des actifs selon l'invention est telle qu'elle est compatible avec la destination d'un article absorbant selon l'invention. En d'autres termes, les actifs sont réalisées dans un matériau de préférence inerte à l'égard de l'utilisateur, afin d'éviter tout effet indésirable tel que par exemples des phénomènes d'allergies et/ou d'irritations. L'élément limitatif est avant tout la compatibilité de l'actif avec la technique d'encapsulation retenue.

Ainsi, l'actif microencapsulé selon l'invention présente avantageusement une solubilité dans l'eau à 20 °C inférieure à 4 %, de préférence inférieure à 1 %.

De préférence, l'actif microencapsulé peut être présent au sein des microcapsules selon l'invention en une teneur comprise entre 10 % et 40 % en poids, de préférence entre 25 % et 35 % en poids, par rapport au poids total de la suspension de microcapsules.

A titre illustratif et non limitatif des actifs pouvant être considérés selon l'invention peuvent notamment être cités les actifs odorants, les actifs désodorisants, les actifs cosmétiques, les actifs désinfectants, les actifs bactéricides, et leurs mélanges.

De préférence, l'actif cosmétique peut être choisi parmi les actifs hydratants, les actifs apaisants, les actifs anti-irritants, et leurs mélanges.

Selon un mode de réalisation particulier, l'actif est un actif odorant ou désodorisant, en particulier un parfum.

Selon un autre mode de réalisation particulier, l'actif mis en oeuvre dans les microcapsules selon l'invention peut être un actif anti-irritant.

Ce mode de réalisation est notamment avantageux dans les cas des couches culottes pour enfant dont la peau est particulièrement sensible et réactive.

Selon encore un autre mode de réalisation particulier, l'actif mis en oeuvre dans les microcapsules selon l'invention peut être un actif hydratant ou apaisant, comme par exemple les huiles, les dérivés de karité, les dérivés d'avocat, les vitamines et leurs mélanges.

Selon encore un autre mode de réalisation, l'actif mis en oeuvre dans les microcapsules selon l'invention peut être un actif désinfectant, comme par exemple le triclocarban, les dérivés chlorés (par exemple le Dakin®), les dérivés de l'argent et l'argent, et leurs mélanges.

Selon un mode de réalisation, l'actif mis en oeuvre dans les microcapsules selon l'invention peut être un actif volatil, notamment eu égard aux actifs odorants et désodorisants.

Par « volatil », au sens de la présente invention, on entend désigner tout composé dont l'odeur est perceptible dans l'atmosphère environnante (i.e. une distance inférieure à 5 mètres) à température ambiante et pression atmosphérique.

Selon un mode de réalisation particulier, un article absorbant selon l'invention peut comprendre différentes sortes de microcapsules distinctes l'une de l'autre de par la nature de l'actif qu'elles contiennent.

### Milieu aqueux

Selon un mode de réalisation, le milieu aqueux dédié à assurer la dissolution des microcapsules peut être figuré par l'eau.

Selon un mode de réalisation plus préféré, notamment au regard de la destination d'un article absorbant selon l'invention, le milieu aqueux dédié à assurer la dissolution des microcapsules peut être figuré par au moins un fluide et/ou exsudat corporel.

De préférence, le fluide et/ou exsudat corporel peut être figuré par les larmes, le mucus nasal, l'urine, les menstruations, la sueur, les matières fécales, et leurs mélanges.

### Composé additionnel

Selon un mode de réalisation particulier, un article absorbant selon l'invention peut en outre comprendre tout autre actif sous une forme non encapsulée, sous réserve que ces actifs additionnels n'altèrent pas les propriétés attachées aux composés de l'invention cités précédemment.

A titre de tel actif, peut notamment être cité le bicarbonate de sodium, la zéolithe ou les huiles essentielles. Le bicarbonate de sodium est avantageux en ce qu'il favorise le bien être de la peau en neutralisant les modifications de pH consécutives à la présence de fluides corporels et/ou, le cas échéant, de l'actif spécifique microencapsulé.

### Article selon l'invention

De préférence, un article absorbant selon l'invention peut être jetable. Le terme «jetable» est utilisé ici pour décrire des articles absorbants qui ne sont pas destinés à être lavés, restaurés ou réutilisés en tant qu'article absorbant après une seule utilisation.

Les articles absorbants sont de préférence jetables après une seule utilisation.

Comme indiqué précédemment, la présente invention vise un article absorbant avant usage, c'est à dire avant toute mise en contact dudit article absorbant avec le(s) fluide(s) corporel(s) et/ou exsudats excrétés par le corps humain à absorber, comprenant des microcapsules et au moins un agent de libération présents dans ledit article sous une forme isolée l'une de l'autre. Ainsi, un article selon l'invention comprend des microcapsules, d'une part, et au moins un agent de libération, d'autre part.

Ainsi, selon un mode de réalisation particulier, un article absorbant selon l'invention peut être formé d'une unique couche, les microcapsules étant immobilisées sur une des surfaces, de préférence celle la plus en contact avec la peau, et l'agent de libération étant immobilisé sur l'autre surface, de préférence celle la plus en contact avec le milieu extérieur.

Selon un autre mode de réalisation particulier, un article absorbant selon l'invention peut également être formé d'au moins :
- une première couche comprenant au moins l'agent de libération, et
- une seconde couche comprenant au moins les microcapsules.

Selon une variante de réalisation préférée, la seconde couche peut être disposée au-dessus de la première couche. Cet agencement est conditionné par le parcours susceptible d'être effectué par le milieu aqueux qui, pour des raisons évidentes, est pour l'essentiel conditionné par la force de gravité de la terre, voire la force de capillarité des matériaux constituant au moins une des couches de l'article absorbant.

Cette variante de réalisation est en effet particulièrement avantageuse en ce qu'elle conduit à une nécessaire mise en contact entre le milieu aqueux et l'agent de libération avant la mise en contact de ce milieu aqueux avec les microcapsules. Ainsi, le déplacement du milieu aqueux à travers l'article absorbant selon l'invention conduit à un déplacement concomitant de l'agent de libération vers les microcapsules et ainsi, à la réaction conduisant à une libération immédiate, et donc améliorée, notamment en termes de rapidité, de l'actif présent au sein desdites microcapsules.

Selon un autre mode de réalisation, un article absorbant selon l'invention peut être formé d'au moins :
- une première couche comprenant au moins l'agent de libération,
- une seconde couche comprenant au moins les microcapsules, et
en outre d'une couche intermédiaire disposée entre la première et la seconde couche comprenant respectivement l'agent de libération et les microcapsules.

De préférence, la couche intermédiaire est apte à laisser passer les milieux liquides, notamment physiologiques. Pour satisfaire à ce mode de réalisation particulier, la couche intermédiaire est de préférence poreuse.

La couche intermédiaire peut être un voile, de préférence hydrophile, permettant d'isoler à l'état sec les première et seconde couches décrites ci-dessus, mais autorisant leur mise en contact en présence d'un milieu aqueux.

Cependant, et comme indiqué ci-dessus, un article absorbant selon l'invention peut également être figuré par une éponge, un mouchoir ou une lingette.

Un article absorbant selon l'invention, notamment compte-tenu du système en couches considéré ci-dessus, peut avantageusement être multicouches et comprendre notamment une couche supérieure, un noyau absorbant et une couche inférieure (sauf ceux à usage interne tels que des tampons).

La couche supérieure, la couche inférieure et le noyau absorbant peuvent être formés à partir de tous matériaux connus de l'homme de l'art. De tels matériaux sont notamment décrits dans EP 2 468 309.

Par « couche supérieure », on entend désigner la surface de l'article destinée à être portée en direction ou à proximité du corps de l'utilisateur.

Par « couche inférieure », on entend désigner le côté opposé à la couche supérieure, à savoir la surface de l'article destinée à être portée vers ou placée adjacent aux sous-vêtements ou vêtements de l'utilisateur.

La couche inférieure est de préférence imperméable aux liquides, en particulier aux fluides et exsudats corporels et est de préférence fabriquée à partir d'un film plastique mince, bien que d'autres matériaux flexibles imperméables aux liquides peuvent également être utilisés.

Par "flexible", au sens de la présente invention, on entend désigner des matériaux qui sont souples et qui épouseront facilement la forme générale et les contours du corps humain. La couche inférieure est dédiée à empêcher les fluides ou exsudats corporels absorbés et contenus dans le noyau absorbant de mouiller les éléments susceptibles de rentrer en contact avec l'article absorbant tels que les draps, les pantalons, les pyjamas et les sous-vêtements.

La couche inférieure peut avantageusement être perméable à la sueur (textile dit «respirant»), tout en restant imperméable aux autres fluides et/ou exsudats corporels.

Au vu de ce qui précède, la première couche comprenant au moins l'agent de libération peut être figurée par la couche supérieure, le noyau absorbant ou la couche inférieure, de préférence le noyau absorbant ou la couche supérieure, et mieux la couche supérieure.

La seconde couche comprenant au moins les microcapsules peut être figurée par la couche supérieure, le noyau absorbant ou la couche inférieure, de préférence le noyau absorbant.

Dans toute la description, y compris les revendications, l'expression « comportant un » doit être comprise comme étant synonyme de « comportant au moins un », sauf si le contraire est spécifié.

Les expressions « compris entre ... et ... » et « allant de ... à ... » doivent se comprendre bornes incluses, sauf si le contraire est spécifié.

Les exemples qui suivent sont présentés à titre illustratif et non limitatif de l'invention. Les pourcentages sont exprimés en poids de Matières Premières. Les composés sont, selon le cas, cités en noms chimiques ou en noms CTFA (International Cosmetic Ingrédient Dictionary and Handbook).

### EXEMPLES

### Exemple 1 : fabrication de microcapsules conformes à l'invention

Dans un litre d'eau désioniée à 40° C, on introduit sous agitation 55 g de gélatine d'une valeur bloom d'environ 160. On chauffe entre 40 et 50°C jusqu'à dissolution totale de la gélatine, puis on ajoute 18,5 g d'homopolymère d'acide acrylique de poids moléculaire d'environ 1800 (Coatex Gx). On amène le pH à 8 par ajout de soude à 20 %.

On pèse 880 g de parfum Jasmin.

On mélange la phase organique et la phase aqueuse sous agitation et on émulsionne jusqu'à obtention de particules ayant un diamètre moyen compris entre 5 et 6 micromètres.

On mélange ensuite dans un réacteur thermostatique, muni d'un agitateur, l'émulsion obtenue précédemment et une solution de 17 g de carboxyméthylcellulose de viscosité 250 mPas dans 625 ml d'eau désionisée.

La mesure de viscosité est telle que définie dans FR 2 718 059.

On élève la température à 60° C et on ajoute de l'acide acétique de manière à ajuster le pH à 4,3. On refroidit le coacervat à 8°C et on maintient cette température durant 10 heures. Le durcissement des parois est effectué par addition de 17 g de glutaraldéhyde à 50 % sous forte agitation.

On maintient la température à 8°C pendant 16 heures ainsi qu'une bonne agitation avant de remonter le pH à 7 à la température de 20°C.

Les microcapsules obtenues sont parfaitement isolées et ont un extrait sec de 35 % pour une viscosité de 20 mPas seulement.

### Exemple 2 : évaluation de l'effet de tensioactifs comme agent de libération sur la libération d'un actif odorant

Les essais sont réalisés avec des microcapsules G Jasmin décrites en exemple 1.

Quatre tensioactifs de familles chimiques différentes sont testés, à savoir:

| **N°** | **Nom commercial** | **Fournisseur** | **INCI** |
|---|---|---|---|
| **1** | Aromox MCDW | Genencor | Oxyde de Cocodiméthyl amine |
| **2** | Eumulgin HRE 60 | Cognis | huile de ricin hydrogénée PEG-60 |
| **3** | Lutensol AT50 | BASF | Alcools en C₁₆-₁₈ éthoxylé |
| **4** | Sulfopon 1218G | Cognis | Alcool gras en C₁₂-₁₈ sulfaté, sel de sodium |

### Mode opératoire

1-Diluer les microcapsules G Jasmin à 35 % de parfum de l'exemple 1 par deux avec de l'eau du robinet.
2-Pulvériser une quantité définie (environ 1 gramme) de microcapsules diluées sur un mouchoir de type « *tissue* », en veillant à bien répartir les microcapsules sur tout le mouchoir,
3-En parallèle, pulvériser le même poids de tensioactifs à 10 % dans l'eau sur un autre mouchoir de type « *tissue* » en veillant à bien répartir les tensioactifs sur tout le mouchoir,

| | **Tensioactif testé** | **Masse de microcapsules (en g)** | **Masse de tensioactifs (en g)** |
|---|---|---|---|
| **1** | Aromox MCDW | 1,128 | 1,097 |
| **2** | Eumulgin HRE 60 | 1,102 | 1,16 |
| **3** | Lutensol AT50 | 1,113 | 1,181 |
| **4** | Sulfopon 12/186 | 1,069 | 1,047 |

4-Faire sécher à l'air libre les mouchoirs,
5-Assembler les deux mouchoirs de façon à ce que les faces pulvérisées soient en contact et que le mouchoir contenant le tensioactif soit au dessus de celui contenant les microcapsules,
6-Mouiller de façon homogène les mouchoirs avec 4 grammes d'eau et les aplatir à l'aide d'une grille, et
7-Sentir, sans toucher aux mouchoirs, à quelques centimètres de hauteur et effectuer un classement des mouchoirs dégageant le plus de parfum à ceux qui sentent le moins fort, les microcapsules dégageant le plus de parfum étant celles qui réagissent le mieux avec les tensioactifs et l'eau.

### Résultats

On effectue un classement des mouchoirs dans un ordre odorant décroissant.

On obtient le résultat : 4 > 2 ∼ 3 > 1.

En conséquence, l'ensemble des tensioactifs mis en oeuvre dans cet essai permettent une libération très satisfaisante de l'actif odorant considéré, avec cependant un effet amélioré pour les tensioactifs de type Sulfopon 12/186 et Eumulgin HRE 60, et plus particulièrement de type Sulfopon 12/186.

### Exemple 3 : évaluation de l'effet du tensioactif associé à une enzyme comme agent de libération sur la libération d'un actif odorant

Le mode opératoire décrit en exemple 2 est reproduit en considérant en outre la présence d'une enzyme de type protéase (Purafect OX Enzyme, Danisco US Inc. - Genencor Division) qui est ajoutée à hauteur de 5 % dans les solutions préexistantes de tensioactifs à 10 % dans l'eau.

Ces essais sont réalisés avec des microcapsules G Hexanal 5 microns faites en suivant le processus de l'exemple 1, le parfum Jasmin étant remplacé par une solution à 50 % d'hexanal dans du myristate d'isopropyle.

| | **Tensioactif testé** | **Enzyme** | **Masse de microcapsules (en g)** | **Masse de tensioactifs (en g)** |
|---|---|---|---|---|
| 5 | Eumulgin HRE 60 | Non | 1,295 | 1,151 |
| 6 | Eumulgin HRE 60 | Oui | 1,266 | 1,122 |
| 7 | Sulfopon 12/186 | Non | 1,248 | 1,117 |
| 8 | Sulfopon 12/186 | Oui | 1,255 | 1,157 |

### Résultats

On effectue un classement des mouchoirs dans un ordre odorant décroissant.

On obtient le résultat : 8 > 6 > 7 > 5.

Ces résultats confirment que la présence d'une enzyme associée à un tensioactif rend les microcapsules encore plus sensibles à l'eau. Qui plus est, ces résultats confirment que la présence d'un tensioactif permet une libération très satisfaisante de l'actif odorant considéré, avec un effet encore amélioré pour le tensioactif Sulfopon par rapport au tensioactif Eumulgin.

### Exemple 4 : évaluation de l'effet des microcapsules sur la libération d'un actif odorant

Le mode opératoire décrit en exemple 3 est reproduit. Le tensioactif considéré est le Sulfopon 12/186. Les microcapsules sont celles identifiées dans le tableau et les références (1) à (4) ci-après.

| **Type de microcapsules** | **Parfum** | **Diamètre (en µm)** | **Masse de tensioactifs (en g)** | **Masse de microcapsules (en g)** |
|---|---|---|---|---|
| **Gélatine (1)** | Fleur de lin | 5 | 1,332 | 1,335 |
| **Gélatine (2)** | Fleur de lin | 6 | 1,326 | 1,260 |
| **Silicone (3)** | Fleur de lin | 4 | 1,242 | 1,241 |
| **Mélamine (4)** | Fleur de lin | 6 | 1,326 | 1,266 |

| | | | | |
|---|---|---|---|---|
| (1) Microcapsules décrites en exemple 1 de FR 2 718 059 (étape de réticulation limitée au glutaraldéhyde. Plus précisément, seconde étape utilisant l'alun de chrome non réitérée). (2) Microcapsules décrites en exemple 1 de FR 2 718 059. (3) Microcapsules décrites en exemple 3 de FR 08 57111. (4) Microcapsules décrites en exemple 1 de US 4,406,816 dans lequel un parfum remplace la phase interne décrite. | | | | |

### Résultats

On effectue un classement des mouchoirs dans un ordre odorant décroissant.

On obtient les résultats suivants : (1) >(2)>> (3) > (4).

Les microcapsules de mélamine et silicone ne sentent rien. Elles ne sont donc pas du tout sensibles à l'eau et ce, même en présence du tensioactif et de l'enzyme.

En revanche, les microcapsules de gélatine sont particulièrement satisfaisantes pour assurer une libération rapide de l'actif odorant considéré.

## Revendications

1. Article absorbant comprenant, sous une forme immobilisée :
a) des microcapsules formées à raison d'au moins 25 % en poids par rapport au poids total en polymère(s) formant les microcapsules d'un matériau dérivant d'au moins un polymère naturel, d'un de ses analogues protéiniques, ou d'un de ses dérivés, et chargées en au moins un actif, et
b) au moins un agent de libération, apte à stimuler la dissolution des microcapsules lors de la mise en contact de l'article absorbant avec un milieu aqueux, et choisi parmi les enzymes, les tensioactifs hydrosolubles à température ambiante et pression atmosphérique, et leurs mélanges,
les microcapsules a) et l'agent de libération b) étant présents dans ledit article sous une forme isolée l'une de l'autre.

2. Article selon la revendication 1, **caractérisé en ce que** les microcapsules sont obtenues au moyen d'une technique de microencapsulation par coacervation complexe d'au moins un polymère naturel amphotère ou cationisable et d'au moins un polymère portant des charges anioniques.

3. Article selon la revendication 2, **caractérisé en ce que** la technique de microencapsulation par coacervation complexe comprend en outre, postérieurement à l'étape de coarcervation, une étape de réticulation via un aldéhyde, de préférence du glutaraldéhyde, l'aldéhyde représentant de préférence de 5 % à 30 % en poids, préférentiellement de 10 à 20 % en poids, par rapport au poids total du ou des polymères naturels amphotères ou cationisables.

4. Article selon la revendication 2 ou 3, **caractérisé en ce que** le ou les polymères naturels amphotères ou cationisables sont choisis parmi la gélatine, la chitine, un de leurs analogues protéiniques, ou un de leurs dérivés, et leurs mélanges, de préférence la gélatine.

5. Article selon l'une quelconque des revendications 2 à 4, **caractérisé en ce que** le ou les polymères portant des charges anioniques sont choisis parmi l'acide polyacrylique, la carboxyméthylcellulose, les alginates, les polyacides synthétiques, la gomme arabique et leurs mélanges.

6. Article selon l'une quelconque des revendications précédentes, **caractérisé en ce que** le matériau dérivant d'au moins un polymère naturel, d'un de ses analogues protéiniques, ou d'un de ses dérivés, forme les microcapsules à raison d'au moins 50 % en poids, de préférence d'au moins 60 % en poids, plus particulièrement d'au moins 70 % en poids, et mieux d'au moins 80 % en poids, par rapport au poids total en polymères formant lesdites microcapsules.

7. Article selon l'une quelconque des revendications précédentes, **caractérisé en ce que** l'actif microencapsulé représente une teneur comprise entre 10 % et 40 % en poids, de préférence entre 25 % et 35 % en poids, par rapport au poids total de la suspension de microcapsules.

8. Article selon l'une quelconque des revendications précédentes, **caractérisé en ce que** l'agent de libération est choisi parmi les tensioactifs non ioniques, anioniques, cationiques ou amphotères, et leurs mélanges.

9. Article selon l'une quelconque des revendications précédentes, **caractérisé en ce que** l'agent de libération est figuré par au moins un tensioactif hydrosoluble à température ambiante et pression atmosphérique choisi parmi l'oxyde de cocodiméthyl amine, l'huile de ricin hydrogénée PEG-60, les alcools en C16-18 éthoxylés, les alcools gras en C12-18 sulfatés, et leurs mélanges, de préférence parmi l'huile de ricin hydrogénée PEG-60 et les alcools gras en C12-18 sulfatés, et mieux les alcools gras en C12-18 sulfatés.

10. Article selon l'une quelconque des revendications précédentes, **caractérisé en ce que** l'agent de libération est figuré par au moins une enzyme choisie parmi les protéases lorsque le polymère naturel est figuré par une protéine, en particulier la gélatine, les amylases lorsque le polymère naturel est figuré par un polysaccharide, en particulier l'amidon, et leurs mélanges, de préférence est figurée par Purafect OX Ensyme.

11. Article selon l'une quelconque des revendications précédentes, **caractérisé en ce que** le milieu aqueux est figuré par au moins de l'eau ou un fluide et/ou exsudat corporel, de préférence choisi parmi les larmes, le mucus nasal, l'urine, les menstruations, la sueur, les matières fécales, et leurs mélanges.

12. Article selon l'une quelconque des revendications précédentes, **caractérisé en ce que** l'actif microencapsulé est choisi parmi au moins un actif odorant, en particulier un parfum, un actif désodorisant, un actif désinfectant, un actif bactéricide, un actif cosmétique, et leurs mélanges; l'actif cosmétique étant de préférence choisi parmi au moins un actif hydratant, un actif apaisant, un actif anti-irritant, et leurs mélanges.

13. Article selon l'une quelconque des revendications précédentes, **caractérisé en ce que** ledit article est formé d'au moins :
- une première couche comprenant au moins l'agent de libération,
- une seconde couche comprenant au moins les microcapsules, de préférence la seconde couche étant disposée au-dessus de la première couche.

14. Article selon la revendication 13, **caractérisé en ce que** ledit article comprend en outre au moins une couche intermédiaire disposée entre la première et la seconde couche, de préférence ladite couche intermédiaire étant apte à laisser passer les milieux liquides, notamment physiologiques, en particulier au moyen de pores.

15. Article selon l'une quelconque des revendications précédentes, **caractérisé en ce que** ledit article est une serviette hygiénique, un protège-slip, un tampon, un article pour adultes incontinents ou une couche pour bébé.

16. Utilisation d'un article tel que défini selon l'une quelconque des revendications 1 à 15, pour maîtriser et/ou réduire les mauvaises odeurs des fluides corporels et/ou exsudats excrétés par le corps humain, en particulier l'urine et les menstruations.

17. Article tel que défini selon l'une quelconque des revendications 1 à 15, pour son utilisation pour maîtriser et/ou réduire les irritations, inflammations et/ou infections liées audit article et/ou aux fluides corporels et/ou exsudats excrétés par le corps humain.

## Patentansprüche

1. Saugfähiger Artikel, umfassend in immobilisierter Form:
a) Mikrokapseln, die in einer Menge von zumindest 25 Gew.-%, bezogen auf das Gesamtgewicht an Polymere(n), das (die) die Mikrokapseln ausbildet/-n, aus einem Material, das von zumindest einem natürlichen Polymer, einem seiner Proteinanaloga oder einem seiner Derivate abgeleitet ist, ausgebildet und mit zumindest einem Wirkstoff sind, und
b) zumindest ein Freisetzungsmittel, das dazu geeignet ist, die Auflösung der Mikrokapseln zu stimulieren, wenn der saugfähige Artikel mit einem wässrigen Medium in Kontakt gebracht wird, und aus Enzymen, bei Raumtemperatur und atmosphärischem Druck wasserlöslichen Tensiden und deren Gemischen ausgewählt ist,
wobei die Mikrokapseln a) und das Freisetzungsmittel b) im Artikel in voneinander isolierter Form vorliegen.

2. Artikel nach Anspruch 1, **dadurch gekennzeichnet, dass** die Mikrokapseln mittels einer Mikroverkapselungstechnik durch komplexe Koazervierung zumindest eines natürlichen amphoteren oder kationisierbaren Polymers und zumindest eines Polymers mit anionischen Ladungen erhalten werden.

3. Artikel nach Anspruch 2, **dadurch gekennzeichnet, dass** die Mikroverkapselungstechnik durch komplexe Koazervierung ferner nach dem Koazervierungsschritt einen Vernetzungsschritt über ein Aldehyd, vorzugsweise Glutaraldehyd, umfasst, wobei das Aldehyd vorzugsweise 5 bis 30 Gew.-%, bevorzugtermaßen 10 bis 20 Gew.-%, bezogen auf das Gesamtgewicht des bzw. der natürlichen amphoteren oder kationisierbaren Polymers/-e, darstellt.

4. Artikel nach Anspruch 2 oder 3, **dadurch gekennzeichnet, dass** das bzw. die natürliche(n) amphotere(n) oder kationisierbare(n) Polymer(e) aus Gelatine, Chitin, einem ihrer Proteinanaloga oder einem ihrer Derivate und ihren Gemischen, vorzugsweise Gelatine, ausgewählt ist bzw. sind.

5. Artikel nach einem der Ansprüche 2 bis 4, **dadurch gekennzeichnet, dass** das bzw. die Polymer(e) mit anionischen Ladungen aus Polyacrylsäure, Carboxymethylcellulose, Alginaten, synthetischen Polysäuren, Gummiarabikum und deren Gemischen ausgewählt ist bzw. sind.

6. Artikel nach einem der vorangehenden Ansprüche, **dadurch gekennzeichnet, dass** das von zumindest einem natürlichen Polymer, einem seiner Proteinanaloga oder einem seiner Derivate abgeleitete Material die Mikrokapseln in einer Menge von zumindest 50 Gew.-%, vorzugsweise zumindest 60 Gew.-%, insbesondere zumindest 70 Gew.-% und noch besser zumindest 80 Gew.-%, bezogen auf das Gesamtgewicht an Polymeren, welche die Mikrokapseln ausbilden, ausbildet.

7. Artikel nach einem der vorangehenden Ansprüche, **dadurch gekennzeichnet, dass** der mikroverkapselte Wirkstoff einen Gehalt von 10 bis 40 Gew.-%, vorzugsweise 25 bis 35 Gew.-%, bezogen auf das Gesamtgewicht der Mikrokapselsuspension, darstellt.

8. Artikel nach einem der vorangehenden Ansprüche, **dadurch gekennzeichnet, dass** das Freisetzungsmittel aus nichtionischen, anionischen, kationischen oder amphoteren Tensiden und ihren Gemischen ausgebildet ist.

9. Artikel nach einem der vorangehenden Ansprüche, **dadurch gekennzeichnet, dass** das Freisetzungsmittel durch zumindest ein bei Raumtemperatur und atmosphärischem Druck wasserlösliches Tensid dargestellt ist, das aus Kokosdimethylamin, hydriertem Rizinusöl PEG-60, ethoxylierten C16-18-Alkoholen, sulfatierten C12-18-Fettalkoholen und ihren Gemischen, vorzugsweise aus hydriertem Rizinusöl PEG-60 und den sulfatierten C12-18-Fettalkoholen und noch besser den sulfatierten C12-18-Fettalkoholen ausgewählt ist.

10. Artikel nach einem der vorangehenden Ansprüche, **dadurch gekennzeichnet, dass** das Freisetzungsmittel durch zumindest ein Enzym dargestellt ist, das ausgewählt ist aus Proteasen, wenn das natürliche Polymer durch ein Protein, insbesondere Gelatine, dargestellt ist, aus Amylasen, wenn das natürliche Polymer durch ein Polysaccharid, insbesondere Stärke, dargestellt ist, und ihren Gemischen, vorzugsweise durch Purafect OX-Enzym dargestellt ist.

11. Artikel nach einem der vorangehenden Ansprüche, **dadurch gekennzeichnet, dass** das wässrige Medium zumindest durch Wasser oder eine Körperflüssigkeit und/oder -ausscheidung dargestellt ist und vorzugsweise aus Tränen, Nasenschleim, Urin, Menstruationsblut, Schweiß, Fäkalien und ihren Gemischen ausgewählt ist.

12. Artikel nach einem der vorangehenden Ansprüche, **dadurch gekennzeichnet, dass** der mikroverkapselte Wirkstoff aus mindestens einem Duftwirkstoff, insbesondere einem Parfüm, einem desodorierenden Wirkstoff, einem desinfizierenden Wirkstoff, einem bakteriziden Wirkstoff, einem kosmetischen Wirkstoff und deren Gemischen ausgewählt ist, wobei der kosmetische Wirkstoff vorzugsweise aus einem hydrierenden Wirkstoff, einem beruhigenden Wirkstoff, einem reizlindernden Wirkstoff und deren Gemischen ausgewählt ist.

13. Artikel nach einem der vorangehenden Ansprüche, **dadurch gekennzeichnet, dass** der Artikel aus zumindest Folgendem ausgebildet ist:
- einer zumindest das Freisetzungsmittel umfassenden ersten Schicht,
- einer zumindest die Mikrokapseln umfassenden zweiten Schicht, wobei die zweite Schicht vorzugsweise über der ersten Schicht angeordnet ist.

14. Artikel nach Anspruch 13, **dadurch gekennzeichnet, dass** der Artikel ferner zumindest eine zwischen der ersten und der zweiten Schicht angeordnete Zwischenschicht umfasst, wobei die Zwischenschicht vorzugsweise dazu geeignet ist, flüssige, insbesondere physiologische Medien hindurchzulassen, insbesondere mittels Poren.

15. Artikel nach einem der vorangehenden Ansprüche, **dadurch gekennzeichnet, dass** der Artikel eine Damenbinde, eine Slipeinlage, ein Tampon, ein Artikel für inkontinente Erwachsene oder eine Babywindel ist.

16. Gebrauch eines Artikels nach einem der Ansprüche 1 bis 15 zum Kontrollieren und/oder Reduzieren schlechter Gerüche der Körperflüssigkeiten und/oder Ausscheidungen des menschlichen Körpers, insbesondere Urin und Menstruationsblut.

17. Artikel nach einem der Ansprüche 1 bis 15 für dessen Gebrauch zum Kontrollieren und/oder Reduzieren der Reizungen, Entzündungen und/oder Infektionen, die mit dem Artikel und/oder mit den Körperflüssigkeiten und/oder Ausscheidungen des menschlichen Körpers im Zusammenhang stehen.

## Claims

1. Absorbent article comprising, in an immobilized form:
a) microcapsules formed, in a proportion of at least 25% by weight relative to the total weight of polymer(s) forming the microcapsules, of a material derived from at least one natural polymer, a protein analogue thereof, or a derivative thereof, and said microcapsules being charged with at least one active agent, and
b) at least one release agent that is capable of stimulating the dissolution of the microcapsules during the placing of the absorbent article in contact with an aqueous medium, and selected enzymes, surfactants that are water-soluble at room temperature and atmospheric pressure, and mixtures thereof,
the microcapsules a) and the release agent b) being present in the said article in a form isolated from each other.

2. Article according to Claim 1, wherein the microcapsules are obtained by means of a technique of complex coacervation microencapsulation of at least one amphoteric or cationizable natural polymer and of at least one polymer bearing anionic charges.

3. Article according to Claim 2, wherein the complex coacervation microencapsulation technique further comprises, after the coacervation step, a step of crosslinking via an aldehyde, preferably glutaraldehyde, the aldehyde preferably representing from 5% to 30% by weight, preferably 10 to 20% by weight, relative to the total weight of amphoteric or cationizable natural polymer(s).

4. Article according to Claims 2 or 3, wherein the amphoteric or cationizable natural polymer(s) are selected from gelatin, chitin, a protein analogue thereof, or a derivative thereof, and mixtures thereof.

5. Article according to Claims 2 to 4, wherein the polymer(s) bearing anionic charges are selected from polyacrylic acid, carboxymethylcellulose, alginates, synthetic polyacids, arabic gum, and mixtures thereof.

6. Article according to any one of the preceding claims, wherein the material derived from at least one natural polymer, a protein analogue thereof, or a derivative thereof, forms the microcapsules to a proportion of at least 50% by weight, preferably at least 60% by weight, more preferably at least 70% by weight, and best at least 80% by weight, relative to the total weight of polymer(s) forming the said microcapsules.

7. Article according to any one of the preceding claims, wherein the microencapsulated active agent represents a content of between 10% and 40% by weight, preferably of between 25% and 35% by weight, relative to the total weight of the microcapsule suspension.

8. Article according to any one of the preceding claims, wherein the release agent is selected from nonionic, anionic, cationic and amphoteric surfactants, and mixtures thereof.

9. Article according to any one of the preceding claims, wherein the release agent is featured by at least one surfactant that is water-soluble at room temperature and atmospheric pressure, selected from cocodimethylamine oxide, hydrogenated castor oil PEG-60, ethoxylated C₁₆₋₁₈ alcohols, C₁₂₋₁₈ fatty alcohol sulfates, and mixtures thereof.

10. Article according to any one of the preceding claims, wherein the release agent is featured by at least one enzyme selected from proteases when the natural polymer is featured by a protein, in particular gelatine, amylases when the natural polymer is featured by a polysaccharide, in particular starch, and mixtures thereof, preferably is features by Purafect OX Ensyme.

11. Article according to any one of the preceding claims, wherein the aqueous medium is featured by at least water or a bodily fluid and/or bodily exudates, preferably chosen from tears, nasal mucus, urine, sweat, menstruations, faecal matter, and mixtures thereof.

12. Article according to any one of the preceding claims, wherein the microencapsulated active agent is selected from at least one odorous active agent, in particular a perfume, a deodorizing active agent, a disinfecting active agent, a bactericide active agent, a cosmetic active agent, and mixtures thereof, said cosmetic active agent being preferably chosen from at least one moisturizing active agent, a calmative active agent, an anti-irritant active agent, and mixtures thereof..

13. Article according to any one of the preceding claims, wherein the said article is formed from at least:
- a first layer comprising at least the release agent, and
- a second layer comprising at least the microcapsules, preferably the second layer is arranged above the first layer.

14. Article according to claim 13, wherein the said article further comprises at least one intermediate layer arranged between the first and the second layer, the said intermediate layer preferably being capable of allowing the passage of liquid media, especially physiological media, in particular by means of pores.

15. Article according to any one of the preceding claims, wherein the said article is a sanitary towel, a pantiliner, a tampon, an article for incontinent adults or a baby nappy.

16. Use of an article as defined according to any one of the preceding claims for controlling and/or reducing the unpleasant odours of bodily fluids and/or exudates excreted by the human body, in particular urine and menstruations.

17. Article as defined according to any one of claims 1 to 15, for its use for controlling and/or reducing irritations, inflammations and/or infections associated with said absorbent article and/or with the bodily fluids and/or exudates excreted by the human body.
